(19) Europäisches Patentamt
European Patent Office
Office européen des brevets

(11) EP 0 882 012 B1

(12) FASCICULE DE BREVET EUROPEEN

(45) Date de publication et mention de la délivrance du brevet:
**29.08.2001 Bulletin 2001/35**

(21) Numéro de dépôt: **96944100.5**

(22) Date de dépôt: **27.12.1996**

(51) Int Cl.[7]: **C07C 253/14**

(86) Numéro de dépôt international:
**PCT/FR96/02100**

(87) Numéro de publication internationale:
**WO 97/24318 (10.07.1997 Gazette 1997/30)**

# (54) PROCEDE DE SYNTHESE D'UN DERIVE AROMATIQUE ORTHODI-SUBSTITUE PAR UN ATOME D'HALOGENE AUTRE QUE LE FLUOR ET PAR UN GROUPE CYANO

VERFAHREN ZUR HERSTELLUNG VON AROMATISCHEN DERIVATEN DIE ORTHO-DISUBSTITUIERT SIND DURCH EIN HALOGENATOM ANDERS ALS FLUOR, UND DURCH EINE CYANOGRUPPE

METHOD FOR SYNTHESIS OF AN AROMATIC DERIVATIVE SUBSTITUTED BY A HALOGEN ATOM OTHER THAN FLUORINE AND BY A CYANO GROUP

(84) Etats contractants désignés:
**AT BE CH DE DK ES FI FR GB GR IE IT LI LU MC NL PT SE**

(30) Priorité: **28.12.1995 FR 9515676**

(43) Date de publication de la demande:
**09.12.1998 Bulletin 1998/50**

(73) Titulaire: **RHODIA CHIMIE**
**92408 Courbevoie Cédex (FR)**

(72) Inventeurs:
- **RUSSEL, James**
  **F-30340 Rousson (FR)**
- **GILBERT, Laurent**
  **F-75015 Paris (FR)**
- **MAESTRO, Jean-Pierre**
  **F-39360 Saint-Symphorien-d'Ozon (FR)**

(74) Mandataire: **Hirsch, Denise et al**
**Cabinet Lavoix**
**2, Place d'Estienne d'Orves**
**75441 Paris Cedex 09 (FR)**

(56) Documents cités:
**EP-A- 0 015 427** **EP-A- 0 097 357**
**EP-A- 0 608 713** **FR-A- 2 353 516**

- **CHEMISTRY AND INDUSTRY, 21 Janvier 1978, pages 56-63, XP000601110 WILLIAM PRESCOTT: "The halogen exchange process for the production of fluoro-aromatics" cité dans la demande**
- **JOURNAL OF FLUORINE CHEMISTRY, vol. 61, 1993, pages 193-216, XP000601952 V.M. VLASOV: "Flouride ion as a nucleophile and a leaving group in aromatic nucleophilic substitution reactions"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un procédé de synthèse d'un composé aromatique présentant un noyau aromatique comportant au moins un chaînon C-C substitué sur un atome de carbone par un groupe cyano, et sur l'autre atome de carbone par un atome d'halogène autre que le fluor.

La cyanuration de composés aromatiques halogénés est connue, la réaction la plus employée étant la réaction de Rosenmund von Braun, qui réalise la cyanuration d'aromatiques chlorés ou bromés en présence d'une quantité stoechiométrique d'ions cuivreux. K.W. Rosenmund and E. Struck, Ber. 52, 1749 (1916); J. Von Braun and G. Manz, Ann. 488, 111 (1931).

L'application industrielle de cette réaction est toutefois très lourde et coûteuse, car elle nécessite des opérations de traitement des effluents en vue de la séparation de sels de cuivre, et d'un éventuel recyclage du métal de transition.

Par ailleurs, lorsque le produit visé est un composé aromatique portant un groupe cyano en ortho d'un substituant halogène, la réaction à réaliser est une monocyanuration de l'aromatique ortho dihalogéné correspondant. Or, le greffage d'une fonction nitrile en ortho d'un halogène a tendance à favoriser une deuxième substitution pour conduire à un dicyano, ce qui est en défaveur du rendement de la réaction.

En outre, de façon générale, la réaction de Rosenmund von Braun catalysée au cuivre n'est pas régiosélective vis-à-vis d'un atome d'halogène particulier. Cependant, on observe souvent une substitution régiosélective d'un halogène lorsque ce dernier est en ortho d'un substituant électroattracteur à effet mésomère prépondérant, tel que $NO_2$. Par contre, dans le cas de composés aromatiques à noyau appauvri en électrons par une fonction électroattractrice à effet inductif prépondérant, l'orientation de la substitution n'est pas très nette, bien moins que pour des noyaux appauvris par une fonction électroattractrice par effet mésomère, et des problèmes de régiosélectivité peuvent se poser.

Le but de la présente invention est d'obvier à ces inconvénients et de proposer un procédé de synthèse d'un tel dérivé aromatique disubstitué par un groupe cyano et un atome d'halogène disposés en position ortho, qui mette en oeuvre des réactifs simples et peu coûteux, ne nécessite pas d'opération de traitement des effluents et qui conduise au produit désiré avec un bon rendement et une bonne sélectivité.

EP 608 713 décrit la substitution d'un atome de fluor d'un cycle aromatique appauvri en électrons en ortho par rapport à un second atome de fluor par un atome de chlore.

FR 2 353 516 décrit la substitution d'un atome de chlore d'un cycle aromatique appauvri en électrons en para d'un groupe nitro par un atome de fluor, en présence de fluorure de potassium.

De manière surprenante, les présents inventeurs ont constaté que ce but, ainsi que d'autres qui apparaîtront par la suite, peut être atteint par un procédé en deux étapes avec un grand succès, tant du point de vue rendement que du point de vue de la sélectivité, par rapport au procédé classique à une étape.

A cet égard, l'invention a pour objet un procédé de synthèse d'un composé de formule (I)

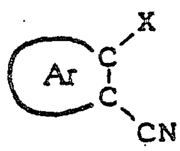

(I)

dans laquelle

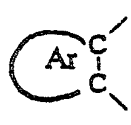

désigne un noyau aromatique appauvri en électrons, éventuellement substitué, contenant au moins cinq chaînons carbonés,

et X est choisi parmi Cl, Br et I,

caractérisé en ce qu'il comprend au moins les étapes consistant à

(a) faire réagir un composé de formule (II)

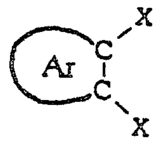

(II)

dans laquelle

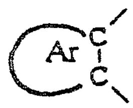

a la signification précédente

et les substituants X, identiques ou différents, sont choisis parmi Cl, Br et I,

avec un réactif d'échange halogène/fluor à base de fluorure pour former majoritairement un composé de formule (III)

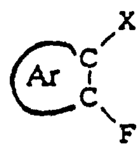

(III)

et

(b) faire réagir le produit réactionnel de l'étape (a) avec un réactif d'échange fluor/cyanure à base de cyanure pour former sélectivement le composé de formule (I).

De manière générale, la réaction de l'étape (a) conduit à un isomère majoritaire de formule (III) et éventuellement à un isomère minoritaire de formule (IIIb),

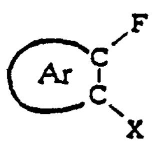

(IIIb)

par monosubstitution par un atome de fluor. On peut même observer une très bonne régiosélectivité selon la nature du composé de formule (II), en particulier en fonction d'éventuels autres substituants du noyau aromatique et de la position de ces derniers. Cela sera détaillé par la suite.

Le produit réactionnel de l'étape (a) peut comprendre cependant un mélange d'isomères dont la séparation est techniquement très difficile et coûteuse à mettre en oeuvre à l'échelle industrielle.

De façon surprenante, la réaction de l'étape (b) fait preuve d'une très forte chimiosélectivité qui amplifie la sélectivité de la séquence synthétique totale en faveur du produit de cyanuration de l'isomère de formule (III), et qui défavorise la cyanuration de l'isomère de formule (IIIb). Ainsi, l'étape (b) peut être conduite sur le mélange réactionnel de l'étape (a) sans séparation des isomères.

En outre, le produit de la double cyanuration du composé de formule (III) n'est pas observé et on obtient le produit désiré avec une très bonne sélectivité.

Le procédé de l'invention s'applique de préférence à des composés de formule (II) où les deux substituants X sont identiques. Avantageusement, chaque X représente un atome de chlore.

De manière générale, il est préférable qu'au moins un X soit un atome de chlore.

Le noyau aromatique

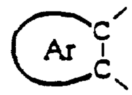

comporte avantageusement 6 chaînons, et est notamment un noyau benzénique. Il peut également comprendre au moins un hétéroatome.

Les éventuels substituants du noyau aromatique peuvent être de nature très variée, par exemple porteurs de fonctions utiles dans la suite de la séquence synthétique. Il peut également s'agir de groupes alkyle, alcényle, alcynyle ou aryle, éventuellement substitués, ou encore de groupes alcénylène ou alcynylène reliés au noyau aromatique pour former un système polycyclique comprenant au moins un noyau aromatique. On peut citer comme exemple de tel noyau un noyau naphtalénique.

Le noyau aromatique du composé de formule (II) est appauvri en électrons du fait de la présence des deux substituants X. Il peut être en outre appauvri en électrons par une fonction électroattractrice formant un chaînon dudit noyau ou par la présence d'au moins un substituant porteur d'une fonction électroattractrice greffé sur un atome dudit noyau, ou par ces deux causes à la fois.

En particulier, dans la formule (II),

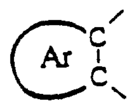

peut désigner un noyau aromatique comprenant au moins un hétéroatome qui appauvrit en électrons le noyau aromatique, tel que notamment l'azote ou le phosphore.

Comme exemples de tels noyaux aromatiques, on peut citer la pyridine ou la quinoléine.

L'appauvrissement en électrons peut également résulter de la substitution dudit noyau par au moins un groupe électroattracteur.

Un tel substituant électroattracteur peut être choisi parmi les groupes attracteurs par effet inductif ou par effet mésomère, tels que définis dans l'ouvrage de référence en Chimie Organique "Advanced Organic Chemistry" par M. J. MARCH, Sème édition, Ed. WILLEY, 1985, notamment pages 17 et 238. On préfère en particulier éviter les groupes portant un atome d'hydrogène susceptible de former des liaisons hydrogène, tels qu'un groupe acide carboxylique, et les groupes portant un atome d'hydrogène basique pouvant conduire à une déprotonation, tels qu'un groupe alkylcétone ayant un hydrogène en a du carbonyle.

Comme exemple de groupes électroattracteurs convenables, on peut citer les groupes $-NO_2$ ; -CN ; $-CF_2R$ où R désigne un atome de fluor ou un radical hydrocarboné; -COOR' où R' désigne un radical hydrocarboné ; CHO ; - Cl ; ou -Br.

Le procédé de l'invention s'applique particulièrement avantageusement à des composés de formule (II) dont le noyau aromatique appauvri en électrons porte un substituant électroattracteur à effet inductif prédominant, ces composés donnant de mauvais résultats dans la réaction directe de type Rosenmund von Braun.

De préférence, un tel substituant est choisi parmi les groupes -CN et $-CF_2R$ où R est choisi parmi un atome de fluor et des groupes hydrocarbonés, avantageusement en $C_{1-20}$, de préférence électroattracteurs eux-mêmes. Ces groupes hydrocarbonés ne comportent avantageusement pas d'atome d'hydrogène basique pouvant conduire à une déprotonation.

Un substituant de formule $-CY_2R$, où Y identiques ou différents sont choisis parmi Cl et F, et R a la signification précédente ou est un atome de chlore peut également être utilisé mais est susceptible de réagir à l'étape (a) pour conduire à un échange chlore/fluor.

Avantageusement, ledit noyau aromatique appauvri en électrons ne porte qu'un seul substituant électroattracteur. De préférence, sur un noyau aromatique à 6 chaînons, ce substituant se trouve en position ortho ou para par rapport à l'atome X qui est destiné à être substitué dans un premier temps par un atome de fluor à l'étape (a) pour former le produit majoritaire de formule (III). La présence d'un tel groupe électroattracteur en ortho ou para de cet atome X a pour effet d'augmenter la sélectivité de la réaction de substitution de l'étape (a) de façon que le produit de formule (III) soit largement majoritaire par rapport au produit de formule (IIIb) selon les réactions suivantes :

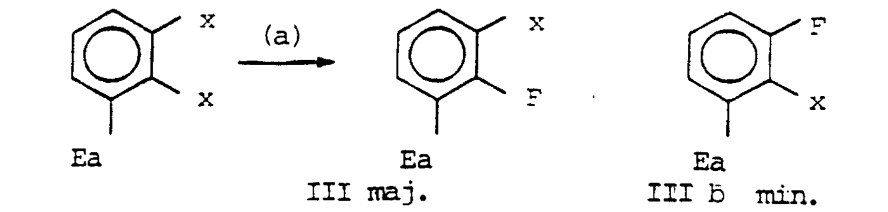


III maj. III b min.

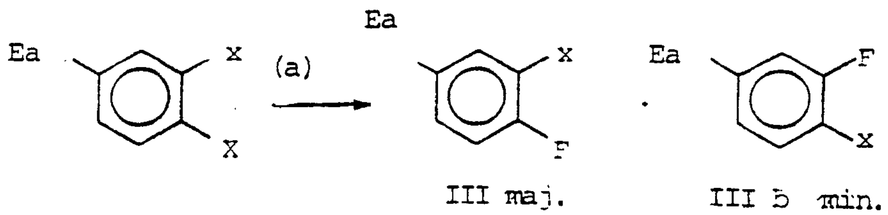


III maj. III b min.

De préférence, lorsque ledit noyau aromatique est un aryle, sa densité électronique est au plus voisine de celle d'un halogénobenzène, notamment d'un dichlorobenzène.

Pour obtenir un appauvrissement en électrons satisfaisant, il est préférable que la somme des constantes de Hammett des fonctions électroattractrices portées par ledit noyau aromatique, soit en tant que chaînon, soit en tant que substituant dudit noyau, soit comprise entre 0,10 et 1,60.

Plus particulièrement, il est avantageux que la somme des constantes de Hammett des substituants dudit noyau aromatique soit comprise entre 0,4 et 1, avantageusement de 0,5 à 0,8.

Il est en outre avantageux que chaque substituant autre que X ait une constante de Hammett comprise entre 0,2 et 0,7.

Pour la définition des constantes de Hammett, on se reportera à l'ouvrage de référence March, "Advanced Organic Chemistry", troisième édition, John Wiley and Son, pages 242 à 250.

A titre d'exemple, le procédé de l'invention peut être mis en oeuvre avec le 3,4-dichloro trifluorométhylbenzène pour synthétiser le 2-chloro 4-trifluorométhyl benzonitrile, avec un bon rendement et une haute sélectivité, suivant le schéma ci-après.

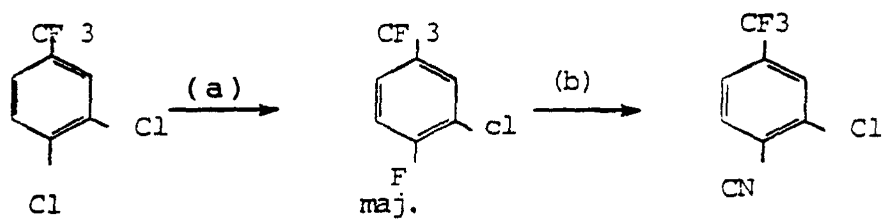


maj.

Le procédé de l'invention comprend essentiellement deux étapes.

Dans un premier temps (a), le composé de formule (II) est amené à réagir avec un réactif d'échange halogène/fluor. Ce réactif est avantageusement un fluorure minéral, de préférence choisi parmi les fluorures de métal alcalin, notamment parmi les fluorures de sodium, de potassium, de rubidium et de césium. Le fluorure de potassium est en général préféré pour des raisons économiques, bien que les fluorures des métaux alcalins de masse atomique supérieure à celle du potassium améliorent sensiblement le rendement de la réaction.

On peut également utiliser comme contre-cation du fluorure, tout cation ayant des propriétés équivalentes à celles d'un cation alcalin, comme par exemple un cation ammonium quaternaire.

De nombreux procédés ont été décrits pour effectuer cette réaction, tels que par exemple ceux décrits dans le certificat d'addition n° 2 353 516 et dans l'article Chem. Ind. (1978)-56, et ont été mis en oeuvre industriellement pour obtenir des fluorures d'aryles, aryles sur lesquels sont greffés des groupements électroattracteurs.

Cette réaction peut se faire par chauffage des partenaires réactionnels à température relativement élevée, généralement de 200 à 280°C, en particulier aux alentours de 250°C, dans un solvant idoine.

En variante, la réaction peut être effectuée à plus basse température, avec une amélioration significative du rendement , en soumettant le milieu réactionnel à l'action d'ultra-sons.

De façon générale, la réaction peut être conduite à une température de 80 à 280°C selon les conditions choisies.

Il convient de signaler que l'utilisation d'ultra-sons dégage une quantité d'énergie importante au sein du milieu réactionnel, cette énergie se substitue en tout ou partie à l'énergie de chauffage usuellement nécessaire.

Les conditions préférées comprennent une puissance d'ultra-sons émise directement par la paroi assurant cette émission au moins égale à 20 W/cm$^2$, avantageusement à 50 W/cm$^2$, plus avantageusement à 100 W/cm$^2$, de préférence à 200 W/cm$^2$.

Les fréquences utilisables sont celles des appareils du commerce, c'est-à-dire que la fréquence des ultra-sons est avantageusement comprise entre 10 et 100 KHz, de préférence entre 15 et 50. Certaines fréquences donnent des résultats très significativement meilleurs; elles correspondent à celles de résonance du milieu dans les conditions de l'expérience et sont en général couvertes par le spectre d'émission relativement large des appareils du commerce.

L'utilisation des ultra-sons permet de réaliser la réaction à des températures relativement peu élevées, comprises entre 80 et 200°C, de préférence de 100 à 150°C.

De manière générale, la réaction a lieu de préférence dans un solvant aprotique dipolaire. La constante diélectrique relative $\in$ dudit solvant est avantageusement au moins égale à 10, de préférence $\in$ est inférieure ou égale à 100 et supérieure ou égale à 25. On préfère particulièrement les solvants dont l'indice donneur D exprimé par la variation d'enthalpie ($\Delta H$ en kcal/mol) de l'association dudit solvant avec le pentachlorure d'antimoine est de 10 à 50.

Des solvants avantageux sont le diméthylsulfoxyde (DMSO), la diméthylsulfone, le diméthylformamide (DMF), la N-méthylpyrrolidone (NMP), le diméthylacétamide (DMAC) et le sulfolane (tétraméthylènesulfone).

Avantageusement, le réactif d'échange halogène/fluor comprend un solide, restant sous forme de solide dispersé dans le milieu réactionnel, et comprenant un fluorure alcalin, et éventuellement un cation promoteur de la réaction.

Avantageusement, le réactif comporte à titre de promoteur un cation alcalin plus lourd que le potassium. Ce cation alcalin peut être introduit sous forme d'un halogénure et pas nécessairement sous forme d'un fluorure. En général, on introduit le cation alcalin sous forme d'un chlorure. La teneur en cation alcalin représente avantageusement de 1 à 5 %, de préférence de 2 à 3 % en mole du fluorure utilisé.

Le réactif peut comporter à titre de promoteur des agents souvent qualifiés de transfert de phase et qui sont des "oniums". Les oniums représentent en général de 1 à 10 %, de préférence de 2 à 5 % en mole du fluorure.

Les oniums sont choisis dans le groupe des cations formés par les éléments des colonnes V B et VI B (tels que définis dans le tableau de classification périodique des éléments publiés au supplément au Bulletin de la Société Chimique de France en janvier 1966), avec 4 ou 3 chaînes hydrocarbonées.

Parmi les oniums dérivant d'éléments de la colonne VB, les réactifs préférés sont les tétraalcoyl ou tétraaryl ammonium ou phosphonium ne présentant pas d'hydrogène en $\beta$ de l'hétéroatome. Le groupe hydrocarboné comporte avantageusement de 4 à 12 atomes de carbone, de préférence de 4 à 8 atomes de carbone. On peut citer par exemple le tétraméthyl ammonium ou le tétraphényl phosphonium. On peut également utiliser des composés de type alkyl pyridinium. Les oniums dérivant de la colonne VIB sont de préférence dérivés d'éléments de numéro atomique supérieur à celui de l'oxygène.

Le procédé aux ultra-sons décrit précédemment permet d'utiliser avantageusement les tétraalcoyl ammonium, qui sont peu stables à une température supérieure à environ 150°C.

La quantité d'onium représente en général de 1 à 10 %, de préférence de 2 à 5 % de la masse du fluorure minéral.

En général, le rapport molaire entre ledit fluorure alcalin ou d'ammonium et ledit substrat est compris entre 0,8 et 1,5, de préférence aux alentours de 1,25 fois la stoechiométrie. Il peut être avantageux de travailler avec un rapport fluorure/substrat sous-stoechiométrique (donc à conversion incomplète) car on limite ainsi les risques de double réaction d'échange halogène/fluor susceptible de survenir lorsque le composé de formule(I) est en présence d'un large excès de fluor, et l'on forme dans ces conditions le composé de formule (I) avec une excellente sélectivité qui améliore la sélectivité du procédé global.

Il est également souhaitable que la teneur en eau du réactif soit au plus égale à environ 2 % de préférence 1 % par rapport à la masse du réactif.

L'agitation est avantageusement menée de manière que au moins 80 %, de préférence au moins 90 % des solides soient maintenus en suspension par l'agitation.

En fait, il est souhaitable que la plus grande partie des solides soient en suspension dans le milieu réactionnel.

Ainsi, l'agitation pour répondre à cette contrainte, ne doit être ni trop forte pour éviter de plaquer, par des effets de cyclone, une partie trop importante des solides contre la paroi du réacteur, ni trop faible pour être capable de faire entrer en suspension les solides issus de la réaction et les solides utilisés comme source de fluor.

Le produit réactionnel de l'étape (a) peut être engagé dans l'étape (b) dès que l'avancement de la première étape est jugé satisfaisant.

Par "produit réactionnel", on entend le produit de la réaction de substitution d'un atome d'halogène par un

atome de fluor sur le composé de formule (II). Ce produit réactionnel comprend généralement un mélange d'isomères résultants de la substitution de l'un ou de l'autre des atomes X, et une quantité très minoritaire de produit de difluoration résultant de la substitution des deux atomes X, ce produit réactionnel contenant majoritairement le composé de formule (III).

Selon l'invention, la séparation du produit réactionnel des réactifs qui n'ont pas été convertis au cours de l'étape (a) n'est pas nécessaire, car ils ne seront pas affectés par le réactif de l'étape (b), ou bien très peu pour éventuellement fournir du produit de formule (I) désiré. On ne cherche pas non plus à isoler les produits formés, et encore moins les isomères de monofluoration.

Le mélange réactionnel obtenu à la fin de l'étape (a) peut donc être engagé tel quel dans l'étape (b)

De manière générale, le produit réactionnel, éventuellement en solution dans tout ou partie du solvant utilisé pour la réaction de l'étape (a), est engagé à l'état de mélange dans l'étape (b) avec un réactif d'échange fluor/cyanure.

Avantageusement, ce réactif comprend un cyanure choisi parmi les cyanures de métaux des groupes I et II, tels que notamment le sodium, le potassium, le magnésium ou le calcium, et les cyanures d'ammonium quaternaire. Le cyanure de sodium et le cyanure de potassium font partie des réactifs préférés. On peut également utiliser avantageusement un mélange de cyanure de potassium et de chlorure de calcium, source de cyanure de calcium.

De préférence, la quantité de réactif d'échange, exprimée en moles de cyanure, mise en oeuvre à l'étape (b) est comprise entre 1 et 2 fois, avantageusement entre 1 et 1,5 fois, la quantité de composé de formule (2) formé à l'étape (a).

Les partenaires réactionnels sont de préférence mis en présence dans un solvant aprotique polaire. La constante diélectrique relative ∈ dudit solvant est avantageusement au moins égale à 10, de préférence ∈ est inférieure ou égale à 100 et supérieure ou égale à 25. On préfère particulièrement les solvants dont l'indice donneur D (ΔH en kcal/mol de l'association dudit solvant avec le pentachlorure d'antimoine) est de 10 à 50.

La plupart des solvants utilisables à l'étape (b) sont aussi valables pour l'étape (a). C'est ainsi que le produit réactionnel de l'étape (a) peut être mis en jeu dans l'étape (b) dans son solvant réactionnel, ce qui supprime toute opération intermédiaire entre les étapes.

Le solvant utilisable pour l'étape (b) peut sans problème être choisi parmi ceux ayant une constante diélectrique et/ou un indice donneur de l'ordre des limites basses indiquées précédemment, voire inférieur(e) à ces limites (solvants peu ou très peu polaires), comme par exemple l'acétonitrile, le benzonitrile, ou des éthers couronne, en prévoyant l'utilisation complémentaire d'agents de transfert de phase, tels que les "oniums" décrits précédemment.

La température de réaction dépend de la nature du réactif à base de cyanure, et du solvant. De manière générale, une élévation de température accélère la réaction. Avantageusement, on opère entre 20 et 250°C.

La réaction se fait aisément à la pression atmosphérique dans un réacteur classique, mais peut aussi avoir lieu dans un réacteur sous pression, avantageusement à une pression inférieure à $5.10^6$ Pa.

Il est préférable que le milieu réactionnel soit sensiblement anhydre, non seulement pour maintenir la sélectivité de la réaction en faveur de l'isomère désiré, mais aussi pour limiter la corrosion due aux ions fluorure libérés comme sous-produit de la réaction.

La présence d'un métal de transition, notamment de cuivre, dans le milieu réactionnel à l'étape (b) n'est pas avantageuse, car la réaction se fait spontanément. Il est même souhaitable d'opérer avec une teneur en cuivre aussi faible que possible pour éviter les opérations de séparation et de retraitement correspondantes.

De préférence, la teneur en cuivre du système réactionnel de l'étape (b) est $10^{-2}$ mol.$l^{-1}$ ou moins. Avantageusement, cette teneur est inférieure à $10^{-3}$ mol.$l^{-1}$, de préférence à $10^{-4}$ mol.$l^{-1}$.

La teneur en cuivre peut également être exprimée par rapport à la quantité du substrat de la réaction de l'étape (b). Dans ce cas, il est préférable que la quantité de cuivre présente dans le système réactionnel de l'étape (b), exprimée en mol soit inférieure au dixième, avantageusement au centième, tout particulièrement au millième, de la quantité de produit réactionnel mis en oeuvre à l'étape (b), exprimée en mol.

La durée de la réaction est déterminée en fonction de la vitesse de formation de l'isomère final désiré.

Le produit final désiré de formule (I) présent dans le mélange réactionnel à la fin de l'étape (b) peut être isolé aisément. La technique de séparation comprend de préférence les deux opérations suivantes :

- filtration ou distillation pour séparer les composés organiques des sels minéraux,
- distillation fractionnée pour séparer desdits composés organiques le produit désiré de formule (I), les réactifs et produits réactionnels de l'étape (a) n'ayant pas réagi à l'étape (b) et le solvant.

Le procédé selon l'invention peut comprendre les opérations consistant à :

i) mélanger le composé de formule (II) et le réactif d'échange X/F dans un solvant ;
ii) soumettre le mélange ainsi obtenu à un chauffage ou à une émission d'ultra-sons ;
iii) éventuellement séparer la phase solide et/ou au moins une partie du solvant du mélange réactionnel obtenu

en (ii) ;
iv) ajouter au mélange réactionnel de (ii), ou au résidu de l'opération (iii), le réactif d'échange F/CN, avec éventuellement une quantité d'un solvant identique ou différent de celui de l'opération (i) ;
(v) maintenir le contact entre les réactifs en élevant éventuellement la température du mélange ;
(vi) séparer du mélange réactionnel obtenu en v) la phase solide à base de sels minéraux ;
(vii) séparer le composé de formule (I) de la phase liquide restante par distillation fractionnée.

Parmi les sels minéraux séparés en vi), on trouve le réactif de l'opération (i) qui n'a pas réagi et des sels formés par les ions fluorure libérés par la réaction de l'opération (v). Ces sels peuvent avantageusement être recyclés dans l'étape (i) pour y être utilisés comme réactif d'échange halogène/fluor.

Comme on l'a dit précédemment, le procédé en deux étapes selon l'invention fournit le produit désiré avec un très bon rendement. La forte sélectivité de la deuxième étape, qui n'était pas prévisible et a été mise en évidence par les présents inventeurs, permet de traiter directement le mélange réactionnel obtenu à l'étape (a) pour obtenir, à l'issue de l'étape (b), un produit réactionnel comprenant essentiellement le composé de formule (I) désiré.

De fait, le procédé en deux étapes est facile à mettre en oeuvre dans le cadre d'une production à échelle industrielle, dans la mesure où il ne nécessite que des équipements classiques, comprenant si nécessaire une unité de séparation des produits intermédiaires particulièrement simple, et où il permet de s'affranchir des unités de retraitement d'effluents destinées à récupérer les sels de métaux de transition, tels que le cuivre, indispensables dans une installation de production par le procédé en une étape classique.

Par ailleurs, l'étape (b) de cyanuration utilise un réactif économique, d'une part par le faible coût des sels de cyanure, et d'autre part par l'absence de métal de transition.

L'invention va maintenant être illustrée par les exemples suivants qui présentent diverses conditions réactionnelles permettant la synthèse du 2-chloro 4-trifluorométhyl benzonitrile

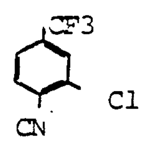

qui est un intermédiaire utile dans la préparation d'herbicides, en deux étapes à partir du 3,4-dichloro trifluorométhyl benzène.

Les résultats présentés dans les exemples sont exprimés en fonction de trois grandeurs qui sont définies ci-après :

- le taux de transformation d'un réactif R (TTR) est le rapport de la quantité (molaire) de R disparue au cours d'une réaction sur la quantité de R initiale ;
- le rendement réel de production d'un produit P à partir d'un réactif R (RRP) est le rapport de la quantité de P produite sur la quantité de R initiale ;
- le rendement de transformation de R en P (RTP) qui est le rapport de la quantité de P produite sur la quantité de R disparue.

**<u>EXEMPLE 1</u>**

<u>A - Synthèse du 3 chloro 4-fluoro-trifluorométhylbenzène</u>

On réalise la réaction suivante :

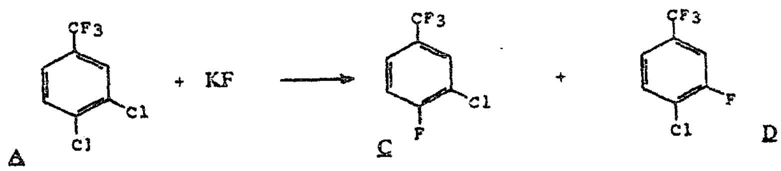

Dans un réacteur en verre, muni d'une agitation mécanique, d'une colonne de fractionnement et d'un réfrigérant, on introduit à température ambiante, en atmosphère anhydre, 35,4 g (0,164 mol) de 3,4-dichlorotrifluorométhyl-

thylbenzène A, 11,6 g (0,200 mol) de fluorure de potassium, 2,8 g (0,008 mol) de bromure de tétra(n-butyl)phosphonium et 44,0 g de sulfolane.

Le mélange réactionnel est chauffé et la température est maintenue à 85 ± 2°C.

La distillation commence après environ 30 minutes et, après une évolution rapide, ralentit progressivement. La réaction est complète au bout de 5 heures.

Le mélange est purifié par distillation fractionnée pour récupérer les fractions aromatiques et le sulfolane.

La conversion de A est TTA = 82,2 %

Le rendement réel en C est RRC = 66,3 %, le rendement de transformation de A en C, RTC vaut 80,7 %.

Le rendement réel en D est RRD = 8,0 %, le rendement de transformation de A en D vaut 9,7 %.

La transformation se fait donc avec une sélectivité à 89 % en faveur de C et à 11 % en faveur de D.

B - Synthèse du 2-chloro 4-trifloromèthyl benzonitrile

Dans un réacteur, on place :

- 1 g du mélange d'isomères C et D séparé en A (1 équivalent molaire)
- 16,1 g de DMSO (41 équivalents molaires)
- 0,52 g de KCN (1,6 " " )

Le mélange réactionnel est chauffé à une température de 60°C pendant 10 h 15 minutes.

L'analyse du mélange réactionnel final permet de déterminer que la réaction correspond à l'équation suivante avec les résultats suivants :

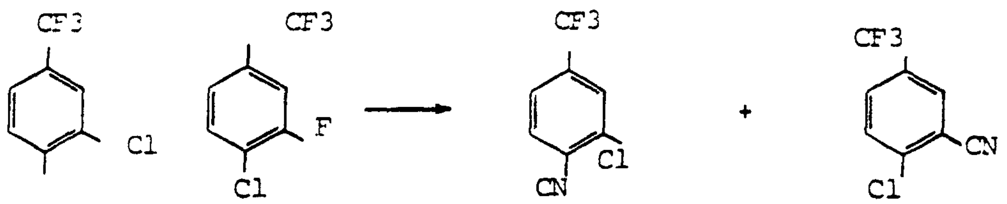

C D E E

2-chloro 4-trifluorométhyl benzonitrile

2-chloro 5-trifluorométhyl benzonitrile

TTC = 94 % ; TTD = 35 % ; RRE = 77 % ; RRF = 11 % ;
RTE 82 % ; RTF = 31 %.

C est converti presque quantitativement (TTC = 94 %) alors que D est peu transformé (TTD = 35%). La réaction est fortement régiosélective.

Par ailleurs, la transformation de C conduit principalement au produit désiré E (RTE = 82 %), alors que la transformation de D ne fournit que peu de produit F d'échange fluor/cyanure (RTE = 31 %).

Par conséquent, la réaction de cyanuration fournit de façon très sélective le produit désiré par rapport à son isomère. En effet, la proportion de l'isomère désiré E par rapport au produit de cyanuration (E et F), exprimé par le rapport molaire

$$\frac{\text{quantité de E produite}}{\text{quantité de E + quantité de F produite}} \quad \text{ou} \quad \frac{E}{E+F}$$

qui est indicatrice de la régio-sélectivité de la réaction, est de 98 %. Le produit désiré étant très faiblement souillé par son isomère dont il est difficile à séparer, il suffit de mettre en oeuvre des techniques de séparation simples pour séparer les autres sous-produits de la réaction et fournir un dérivé chloro cyano trifluorométhylbenzène de haute pureté directement utilisable en vue de transformations ultérieures.

**EXEMPLE 2**

L'étape A est conduite comme dans l'exemple 1 pour obtenir un produit de monofluoration comprenant 89 %

de C et 11 % de D.

L'étape B est conduite en présence de 1,4 équivalents molaires de cyanure de sodium dans du DMSO (27 équivalents molaires) à une température de 90°C pendant 2 h 30 min.

Les résultats de la cyanuration sont présentés dans le tableau 1 suivant qui récapitule les conditions opératoires.

EXEMPLES 3 à 5

Les étapes A et B de l'exemple 1 ont été répétées à de légères variations opératoires près, qui concernent principalement le solvant, la température et la durée de réaction de l'étape B. Les résultats sont présentés dans le tableau 1.

Ces exemples montrent que l'on peut faire varier les conditions opératoires selon les diverses variantes préférées indiquées précédemment en obtenant toujours de très bons résultats en ce qui concerne le rendement et la sélectivité, en particulier si le solvant est le DMF ou le DMSO. Le sulfolane nécessite des conditions réactionnelles un peu plus sévères.

**EXEMPLE 6**

L'étape B de l'exemple 1 a été répétée sur le produit **C** pur (produit de monofluoration comprenant 100 % de C) avec un réactif de cyanuration comprenant un mélange de KCN et de $CaCl_2$ à raison de 1,5 équivalents molaires de KCN et 0,5 équivalent molaire de $CaCl_2$ pour un équivalent molaire de C.

Les résultats de conversion et de transformation sont présentés dans le tableau 1 qui rappelle les conditions opératoires. La conversion est un peu plus faible que dans l'exemple 1, mais le rendement de transformation est plus élevé, de sorte que le rendement de la réaction souhaitée est comparable à celui de l'exemple 1.

**EXEMPLE 7**

L'étape B de l'exemple 1 a été répétée sur le produit C pur avec 1,5 équivalents molaires de cyanure de sodium NaCN dans la N-méthyl pyrrolidone (16 équivalents molaires). Comme le sulfolane, la NMP nécessite des conditions relativement sévères. On obtient toutefois une très bonne conversion et un rendement de réaction satisfaisant.

Les données de cet exemple sont rassemblées dans le tableau 1.

**EXEMPLE 8**

L'étape B de l'exemple 1 a été répétée sur le produit C pur avec 1,9 équivalents molaires de KCN dans l'acétonitrile (11 équivalent molaire) en présence de bromure de tétrabutylammonium en tant qu'agent de transfert de phase (0,08 équivalent molaire).

Les résultats de cet essai, présentés dans le tableau 1 avec les conditions opératoires, sont une fois encore très satisfaisants.

**TABLEAU I**

| Exemple N° | ETAPE A | ETAPE B | | | | | | | | |
|---|---|---|---|---|---|---|---|---|---|---|
| | Rapport C / D | Solvant (éq.) | Réactif (éq.) | Tempé-rature (°C) | Durée (h) | TTC % | TTD % | RRE % | RTE % | $\frac{E}{E+E}$ % |
| 1 | 87/13 | DMSO (41) | KCN (1,6) | 60 | 10,25 | 94 | 35 | 77 | 82 | 98 |
| 2 | 89/11 | DMSO (27) | NaCN (1,4) | 90 | 2,5 | 97 | 27 | 79 | 81 | 98 |
| 3 | 89/11 | DMF (22) | KCN (1,8) | 80 | 13 | 90 | 53 | 71 | 79 | 98 |
| 4 | 90/10 | DMF (37) | KCN (1,7) | 60 | 30 | 85 | 32 | 66 | 80 | 99 |
| 5 | 89/11 | Sulfolane (56) | KCN (1,7) | 100 | 13,5 | 88 | 43 | 65 | 73 | 99 |
| 6 | 100/0 | DMF (32) | KCN (1,5) + $CaCl_2$ (0,5) | 80 | 10,35 | 88 | - | 76 | 86 | - |
| 7 | 100/0 | NMP (16) | NaCN (1,5) | 105 | 4,5 | 96 | - | 70 | 73 | - |
| 8 | 100/0 | MeCN (11) | KCN (1,9) + $Bu_4N^+Br^-$ (0,08) | 82 | 30,0 | 94 | - | 63 | 66 | - |

## Revendications

1. Procédé de synthèse d'un composé de formule (I)

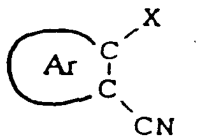 (I)

dans laquelle

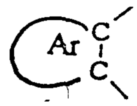

désigne un noyau aromatique à six chaînons appauvri en électrons, éventuellement substitué, contenant au moins cinq chainons carbones

et X est choisi parmi Cl, Br et I,

**caractérisé en ce qu**'il comprend au moins les étapes consistant à :

(a) faire réagir un composé de formule (II)

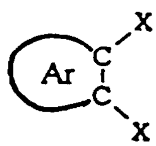 (II)

dans laquelle

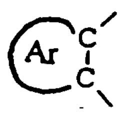

a la signification précédente et les substituants X, identiques ou différents, sont choisis parmi Cl, Br et I, avec un réactif d'échange halogène/fluor à base de fluorure, la réaction aboutissant à un composé de formule (III)

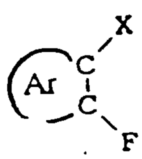 (III)

présent de façon majoritaire dans le milieu réactionnel, et

(b) faire réagir le produit réactionnel de l'étape (a) directement avec un réactif d'échange fluor/cyanure à base de cyanure obtenu de façon sélective, le composé de formule (I) étant alors obtenu de façon sélective.

2. Procédé selon la revendication 1, **caractérisé en ce que** dans le composé de formule (II) les deux substituants X sont identiques.

3. Procédé selon la revendication 2, **caractérisé en ce que** chaque X représente un atome de chlore.

4. Procédé selon l'une quelconque des revendications précédentes, **caractérisé en ce que**, dans le composé de formule (II),

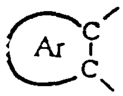

désigne un noyau aromatique comprenant au moins un hétéroatome.

5. Procédé selon l'une quelconque des revendications précédentes, **caractérisé en ce que** ledit noyau aromatique est substitué en outre par au moins un groupe électroattracteur.

6. Procédé selon l'une quelconque des revendications précédentes, **caractérisé en ce que** ledit noyau aromatique est substitué par au moins un groupe électroattracteur à effet inductif prépondérant.

7. Procédé selon la revendication 6, **caractérisé en ce que** ledit groupe électroattracteur par effet inductif est choisi parmi les groupes -CN et $-CF_2R$ où R est choisi parmi un atome de fluor et des groupes hydrocarbonés, de préférence électroattracteurs.

8. Procédé selon l'une quelconque des revendications 5 à 7, **caractérisé en ce que** ledit noyau aromatique est substitué par un seul groupe électroattracteur.

9. Procédé selon les revendications 1 et 8, **caractérisé en ce que** ledit groupe attracteur est en position ortho ou para par rapport à l'atome X à substituer.

10. Procédé selon l'une quelconque des revendications précédentes, **caractérisé en ce que** la somme des constantes de Hammett des fonctions électroattractrices portées par ledit noyau aromatique est comprise entre 0,10 et 1,60.

11. Procédé selon l'une quelconque des revendications précédentes, **caractérisé en ce que** la somme des constantes de Hammett des substituants dudit noyau aromatique est comprise entre 0,4 et 1

12. Procédé selon l'une quelconque des revendications précédentes, **caractérisé en ce que** la constante de Hammett de chacun des substituants dudit noyau aromatique autre que X est comprise entre 0.2 et 0,7.

13. Procédé selon l'une quelconque des revendications précédentes, **caractérisé en ce que** le réactif d'échange halogène/fluor de l'étape (a) est un fluorure minéral, notamment un fluorure de métal alcalin, ou un fluorure d'ammonium quaternaire.

14. Procédé selon l'une quelconque des revendications précédentes, **caractérisé en ce que** la quantité de fluor mise en oeuvre à l'étape (a) est comprise entre 0,8 et 1,5 fois la quantité de composé de formule (I).

15. Procédé selon l'une quelconque des revendications précédentes, **caractérisé en ce que** la réaction de l'étape (a) est conduite à une température de 80 à 280°C.

16. Procédé selon l'une quelconque des revendications précédentes, **caractérisé en ce que** le mélange réactionnel obtenu à la fin de l'étape (a) est engagé tel quel dans l'étape (b).

17. Procédé selon l'une quelconque des revendications précédentes, **caractérisé en ce que** ledit réactif d'échange fluor/cyanure de l'étape (b) comprend un cyanure choisi parmi les cyanures de métaux des groupes I et II, et les cyanures d'ammonium quaternaire.

18. Procédé selon l'une quelconque des revendications précédentes, **caractérisé en ce que** la quantité de cyanure mise en oeuvre à l'étape (b) est comprise entre 1 et 1,5 fois la quantité de composé de formule (III) formé à l'étape (a).

19. Procédé selon l'une quelconque des revendications précédentes, **caractérisé en ce que** la teneur en cuivre du milieu réactionnel de l'étape (b) est de $10^{-2}$ $mol.l^{-1}$ ou moins.

20. Procédé selon l'une quelconque des revendications précédentes, **caractérisé en ce que** la quantité de cuivre présente dans le système réactionnel de l'étape (b), exprimée en mol, est inférieure ou égale au dixième de la

quantité de produit réactionnel de l'étape (a) mise en oeuvre à l'étape (b), exprimée en mol.

**21.** Procédé selon l'une quelconque des revendications précédentes, **caractérisé en ce que** l'on isole à l'étape (b) du fluorure libéré par la réaction d'échange fluor/cyanure, et éventuellement du fluorure n'ayant pas réagi à l'étape (a), et on le recycle à l'étape (a) en tant que réactif d'échange halogène/fluor.

**Patentansprüche**

**1.** Verfahren zur Herstellung einer Verbindung mit der Formel (I)

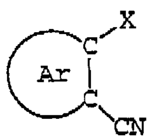

(I),

worin

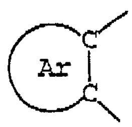

einen gegebenenfalls substituierten, elektronenarmen sechsgliedrigen aromatischen Kern, der mindestens fünf kohlenstoffhaltige Glieder enthält, bedeutet und X aus Cl, Br und I ausgewählt ist, **dadurch gekennzeichnet, dass** es wenigstens die Stufen umfasst, die darin bestehen,

a) eine Verbindung mit der Formel (II)

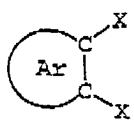

(II),

in welcher

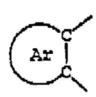

dasselbe wie zuvor bedeutet und die Substituenten X gegebenenfalls verschieden und aus Cl, Br und I ausgewählt sind, mit einem Halogen/Fluor-Austauschreagenz auf der Basis eines Fluorids umzusetzen, wobei die Umsetzung eine Verbindung mit der Formel (III) ergibt

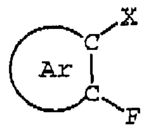

(III),

die überwiegend im Reaktionsmedium vorhanden ist, und

b) das Reaktionsprodukt der Stufe a) direkt mit einem selektiv erhaltenen Fluor/Cyanid-Austauschreagenz auf der Basis eines Cyanids umzusetzen, wobei die Verbindung mit der Formel (I) selektiv erhalten wird.

**2.** Verfahren nach Anspruch 1, **dadurch gekennzeichnet, dass** die beiden Substituenten X der Verbindung mit der Formel (II) gleich sind.

**3.** Verfahren nach Anspruch 2, **dadurch gekennzeichnet**, **dass jedes** X ein Chloratom bedeutet.

**4.** Verfahren nach einem der vorhergehenden Ansprüche, **dadurch gekennzeichnet, dass** in der Verbindung mit der Formel (II)

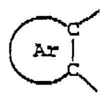

einen mindestens ein Heteroatom enthaltenden aromatischen Kern bedeutet.

**5.** Verfahren nach einem der vorhergehenden Ansprüche, **dadurch gekennzeichnet, dass** der aromatische Kern außerdem durch mindestens eine elektronenanziehende Gruppe substituiert ist.

**6.** Verfahren nach einem der vorhergehenden Ansprüche, **dadurch gekennzeichnet, dass** der aromatische Kern durch mindestens eine elektronenanziehende Gruppe mit vorwiegend induktivem Effekt substituiert ist.

**7.** Verfahren nach Anspruch 6, **dadurch gekennzeichnet, dass** die durch induktiven Effekt elektronenanziehende Gruppe aus den Gruppen -CN und $-CF_2R$ ausgewählt ist, wobei R aus einem Fluoratom und vorzugsweise elektronenanziehenden Kohlenwasserstoffgruppen ausgewählt ist.

**8.** Verfahren nach einem der Ansprüche 5 bis 7, **dadurch gekennzeichnet, dass** der aromatische Kern durch eine einzige elektronenanziehende Gruppe substituiert ist.

**9.** Verfahren nach den Ansprüchen 1 und 8, **dadurch gekennzeichnet, dass** sich die elektronenanziehende Gruppe in Bezug auf das zu substituierende X-Atom in ortho- oder para-Stellung befindet.

**10.** Verfahren nach einem der vorhergehenden Ansprüche, **dadurch gekennzeichnet, dass** die Summe der Hammett-Konstanten der elektronenanziehenden Funktionen, die von dem aromatischen Kern getragen werden, zwischen 0,10 und 1,60 beträgt.

**11.** Verfahren nach einem der vorhergehenden Ansprüche, **dadurch gekennzeichnet, dass** die Summe der Hammett-Konstanten der Substituenten des aromatischen Kerns zwischen 0,4 und 1 beträgt.

**12.** Verfahren nach einem der vorhergehenden Ansprüche, **dadurch gekennzeichnet, dass** die Hammett-Konstante jedes anderen Substituenten des aromatischen Kerns als X zwischen 0,2 und 0,7 beträgt.

**13.** Verfahren nach einem der vorhergehenden Ansprüche, **dadurch gekennzeichnet, dass** das Halogen/Fluor-Austauschreagenz der Stufe a) ein anorganisches Fluorid, insbesondere ein Alkalifluorid, oder ein quaternäres Ammoniumfluorid ist.

**14.** Verfahren nach einem der vorhergehenden Ansprüche, **dadurch gekennzeichnet, dass** die in Stufe a) eingesetzte Fluormenge das 0,8- bis 1,5-Fache der Menge der Verbindung mit der Formel (I) beträgt.

**15.** Verfahren nach einem der vorhergehenden Ansprüche, **dadurch gekennzeichnet, dass** die Umsetzung in Stufe a) bei einer Temperatur von 80 bis 280 °C durchgeführt wird.

**16.** Verfahren nach einem der vorhergehenden Ansprüche, **dadurch gekennzeichnet, dass** das nach Stufe a) erhaltene Reaktionsgemisch als solches in Stufe b) eingesetzt wird.

**17.** Verfahren nach einem der vorhergehenden Ansprüche, **dadurch gekennzeichnet, dass** das Fluor/Cyanid-Austauschreagenz der Stufe b) ein Cyanid umfasst, das aus den Cyaniden der Metalle der Gruppen I und II und quaternären Ammoniumcyaniden ausgewählt ist.

**18.** Verfahren nach einem der vorhergehenden Ansprüche, **dadurch gekennzeichnet, dass** die in Stufe b) eingesetzte Cyanidmenge das 1- bis 1,5-Fache der Menge der in Stufe a) gebildeten Verbindung mit der Formel (III) beträgt.

**19.** Verfahren nach einem der vorhergehenden Ansprüche, **dadurch gekennzeichnet, dass** der Kupfergehalt des Reaktionsmediums in Stufe b) höchstens $10^{-2}$ $mol \cdot l^{-1}$ beträgt.

**20.** Verfahren nach einem der vorhergehenden Ansprüche, **dadurch gekennzeichnet, dass** die in Mol angegebene, im Reaktionssystem der Stufe b) vorhandene Kupfermenge kleiner oder gleich einem Zehntel der in Mol angegebenen Menge an Reaktionsprodukt der Stufe a), die in Stufe b) eingesetzt wird, ist.

**21.** Verfahren nach einem der vorhergehenden Ansprüche, **dadurch gekennzeichnet, dass** in Stufe b) das durch die Fluor/Cyanid-Austauschreaktion freigesetzte Fluorid und gegebenenfalls das in Stufe a) nicht umgesetzte Fluorid abgetrennt und als Halogen/Fluor-Austauschreagenz zur Stufe a) rezirkuliert wird.

## Claims

**1.** Method for synthesis of a compound of formula (I)

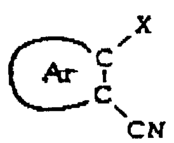

(I)

in which

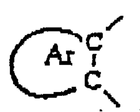

indicates an aromatic ring having six chain links and depleted of electrons, possibly substituted, containing at least five carbon chain links
and X is chosen from Cl, Br and I,
**characterised in that** it includes at least the steps comprising:

a) having a compound of formula (II)

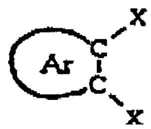

(II)

in which

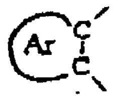

has the preceding meaning and the substituents X, the same or different, are chosen from Cl, Br and I, react with a halogen/fluorine exchange reagent on a basis of fluoride, the reaction resulting in a compound of formula (III)

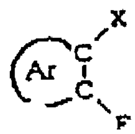

(III)

appearing in the majority in the reaction medium, and

b) having the reaction product of step (a) react directly with a fluorine/cyanide exchange reagent on a basis of cyanide obtained in a selective manner, the compound of formula (I) being thus obtained in a selective manner.

2. Method according to claim 1, **characterised in that** in the compound of formula (II) the two substituents X are the same.

3. Method according to claim 2, **characterised in that** each X represents an atom of chlorine.

4. Method according to any of the preceding claims, **characterised in that**, in the compound of formula (II),

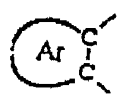

indicates an aromatic ring comprising at least one heteroatom.

5. Method according to any of the preceding claims, **characterised in that** said aromatic ring is furthermore substituted by at least one electroattractor group.

6. Method according to any of the preceding claims, **characterised in that** said aromatic ring is substituted by at least one electroattractor group with a predominantly inductive effect.

7. Method according to claim 6, **characterised in that** said electroattractor group with an inductive effect is chosen from the groups -CN and $-CF_2R$ where R is chosen from an atom of fluorine and hydrocarbon groups, preferably electroattractors.

8. Method according to any of the claims 5 to 7, **characterised in that** said aromatic ring is substituted by a single electroattractor group.

9. Method according to claims 1 and 8, **characterised in that** said attractor group is in ortho or para position with

regard to the atom X to be substituted.

**10.** Method according to any of the preceding claims **characterised in that** the sum of the Hammett constants of the electroattractor functions carried by said aromatic ring is between 0.10 and 1.60.

**11.** Method according to any of the preceding claims, **characterised in that** the sum of the Hammett constants of the substitutents of said aromatic ring is between 0.4 and 1.

**12.** Method according to any of the preceding claims, **characterised in that** the Hammett constant of each of the substituents of said aromatic ring other than X is between 0.2 and 0.7.

**13.** Mathod according to any of the preceding claims, **characterised in that** the halogen/fluorine exchange reagent of step (a) is a mineral fluoride, in particular an alkaline metal fluoride, or a quaternary ammonium fluoride.

**14.** Method according to any of the preceding claims, **characterised in that** the quantity of fluorine used at step (a) is between 0.8 and 1.5 times the quantity of compound of formula (I).

**15.** Method according to any of the preceding claims, **characterised in that** the reaction of step (a) is carried out at a temperature of 80 to 280 °C.

**16.** Method according to any of the preceding claims, **characterised in that** the reaction mixture obtained at the end of step (a) is used as that in step (b).

**17.** Method according to any of the preceding claims, **characterised in that** said fluorine/cyanide exchange reagent of step (b) includes a cyanide chosen from the cyanides of the metals of groups I and II, and the cyanides of quaternary ammonium.

**18.** Method according to any of the preceding claims, **characterised in that** the quantity of cyanide used in step (b) is between 1 and 1.5 times the quantity of compound of formula (III) formed at step (a).

**19.** Method according to any of the preceding claims, **characterised in that** the content of copper of the reaction medium of step (b) is $10^{-2}$ $mol.1^{-1}$ or less.

**20.** Method according to any of the preceding claims, **characterised in that** the quantity of copper present in the reaction system of step (b), expressed in mol, is less than or equal to a tenth of the quantity of reaction product of step (a) used at step (b), expressed in mol.

**21.** Method according to any of the preceding, claims, **characterised in that** fluoride liberated by the fluorine/cyanide exchange reaction, and possibly fluoride which has not reacted at step (a), is isolated at step (b), and is recycled at step (a) as halogen/fluorine exchange reagent.